# EUROPEAN PATENT APPLICATION

(11) **EP 4 659 977 A1**
(43) Date of publication of application: **10.12.2025**
(21) Application number: 24216960.5
(22) Date of filing: 02.12.2024
(51) Int. Cl.: B60K 1/00, B60K 1/02

(54) **CHASSIS, ESPECIALLY FOR HEAVY OFFROAD TRUCK, FITTED WITH DRIVETRAIN**

(30) Priority: 04.12.2023 CZ 20230046
(71) Applicant: TATRA TRUCKS a.s., 742 21 Kopnvnice (CZ)
(72) Inventor: Smolka, Radomír, 74258 P íbor (CZ); Adolt, Luká, 73801 Frýdek-Místek (CZ)
(74) Representative: Havlík, Michal

(57) **Abstract**

Chassis, especially for heavy offroad truck, is fitted with drivetrain, wherein the chassis contains central load carrying tube (207) with at least two axles (6-9) with swinging half-axles, where the chassis has at least one drive unit assigned as part of the drivetrain. The drive unit consists of electric motor (201), with assigned auxiliary gearbox (202), connecting load carrying tube (203) and axis differential (18-21), wherein the drive unit is always assigned to only one axle (6-9).

## Description

### Technical field

The invention relates to a chassis, especially for heavy offroad truck, fitted with drivetrain, wherein the chassis comprises central load carrying tube with at least two axles with swinging half-axles, where at least one drive unit is associated with the chassis as part of the drivetrain.

### Prior Art

The closest prior art is generally known to solve the propulsion using internal combustion engine connected with clutch and main gearbox into a common complex. Using cardan shaft, fixedly connected to flanges on gearbox and supplementary gearbox, is torque transferred to the supplementary gearbox. Or the cardan shaft could be inserted and fixedly connected between the flanges of clutch box and gearbox, which is fixedly connected to the supplementary gearbox. This supplementary gearbox creates in the construction solutions of vehicles of the applicant, together with frame, axles with swinging half-axles, rigid central load-carrying tube and differentials placed in central load-carrying tube, the load-carrying structure of the vehicle. The torque is further transferred into the inter-axle differential and onto axle differentials of rear axles. After connecting the front axle drive, the torque is also transferred onto axle differential of front axles. Next the torque is divided onto individual wheels.

The disadvantage of this solution is the use of internal combustion engine that produces harmful CO₂ and NOₓ emissions, and also higher weight of the drivetrain and higher centre of gravity.

Another known solution according to patent application US2020247224A1 contains electric motor, gearbox, differential box connected into one unit with the frame. But this concept comes from completely different arrangement of the chassis without central load-carrying tube, as it is arranged in construction solutions of the applicant's vehicles. Through driving cardan shafts placed in hollow members, which don't match the arrangement with central load-carrying tube, the torque is transferred onto wheels. Hollow members are connected using a connecting element and together with the electric motor are supported and fixed using support parts.

The disadvantage of this solution compared to the invention is fixed connection between gearbox and differential, which brings higher requirements on installation space around the axle.

In comparison to the presented invention, the need of using a connecting element between the wheels to support the hollow members, in which driving cardan shafts are placed, is also a disadvantage.

Another disadvantage is the fixed connection of frame with electric motor and gearbox. Compared to this, it is also possible to make a chassis with central load-carrying tube as frameless and install a self-supporting body onto it. This eliminates the need for the frame as a load-carrying element.

Cardan driving shafts that have principally lower capacity for transfer of torque are used for non-steered axles, and these are also more expensive in comparison with rigid shafts going through half-axles into the wheel part, used in the presented invention,

Another known solution according to the patent application DE102010024191A1 is a system consisting of gearbox, electric motors, freewheel clutches, drive system, driveshaft, axles and axle gearboxes. But this concept also comes from totally different arrangement of the chassis without central load carrying tube, as it is arranged in the construction solutions of the applicant's vehicles.

Disadvantage of this solution is the use of only two axles with concurrent use of several motors, in comparison with the presented invention, where the number of driven axles is not limited in theory.

This solution uses a driving motor, either internal combustion or electric motor, and auxiliary electric motors connected using freewheel clutches into the gearbox. In the presented invention, in contrast, is always only one electric motor used to drive an axle.

Another disadvantage is the need of using another gearbox between the axles, which results in making the vehicle more expensive, increasing the weight and thus lowering the payload.

Axle gearboxes are fixedly connected with the gearbox, in contrast to this, in the present invention, connection between the axles is not realised, each axle can function independently.

### Summary of the Invention

The object of the invention is to make, especially all-wheel drive, chassis, especially for heavy offroad trucks, based on the chassis with central load-carrying tube and swinging half-axles, with associated electric drive, which would remove at least some of the disadvantages of the above-mentioned state of the art.

All wheel drive chassis generally means chassis with driven wheels of all axles, i.e. with mechanical coupling between the wheel and drive element, i.e. motor.

The object of the invention is solved by a chassis, especially for heavy offroad truck, fitted with drivetrain, wherein the chassis contains central load-carrying tube with at least two axles with swinging half-axles, where the chassis has at least one drive unit associated as part of the drivetrain, wherein according to the invention, the drive unit consist of electric motor, with associated auxiliary gearbox, connecting load-carrying tube and axis differential, wherein the drive unit is always associated with just one axle.

Advantageous embodiment of the invention consists in the electric motor being fixedly connected to the load carrying tube through the auxiliary gearbox

Another advantageous embodiment of the invention consists in the electric motor being oriented with its axis of rotation in the direction of travel.

Another advantageous embodiment of the invention consists in the electric motor being placed in front of or behind the auxiliary gearbox in the direction of travel.

Another advantageous embodiment of the invention consists in the guiding of torque from axis differential into the wheel par using cardan shafts at the steered axles and using rigid shafts going through the half-axles at the non-steered axles.

Another advantageous embodiment of the invention consists in equipping individual axles with autonomous electronic control, independent of the control of the other axles.

In other words, the invention is aimed especially at all wheel drive chassis (wherein it is possible to make a chassis with some axles non-driven), especially for heavy offroad trucks, making use of drive units consisting of electric motor, auxiliary gearbox, connecting load carrying tube and axis differential, driving always only one axle with swinging half-axles, which will be combined with other drive units and central load carrying tube into a complete chassis of a given configuration.

Chassis made of these components has lower weight that the classic arrangement with internal combustion engine. Thanks to the absence of expensive and complex parts, such as internal combustion engine, gearbox, cardan shaft, supplementary gearbox, inter-axle differential, divider (if used in the vehicle), fuel tank, emission and exhaust system, it is also a cheaper solution, both regarding cost of purchase and regarding operating costs connected with repairs and maintenance.

Because the chassis features, in contrast to the prior art, the absence of inter-axle differential and drive shafts, it is further possible to use smaller diameter of the central load carrying tube than in this prior art and thus further decrease the weight and improve the ramp breakover angle.

Another advantage is the modular arrangement, respective modularity of the whole chassis, which allows for easy changing of parameters, such as the wheel base of the vehicle, number of axles or size of the electric motor, which allows for changing the power of the vehicle.

Last, but not least, the chassis solution according to the presented invention features lower weight and simpler construction structure with respectively higher reliability.

### Overview of the Drawings

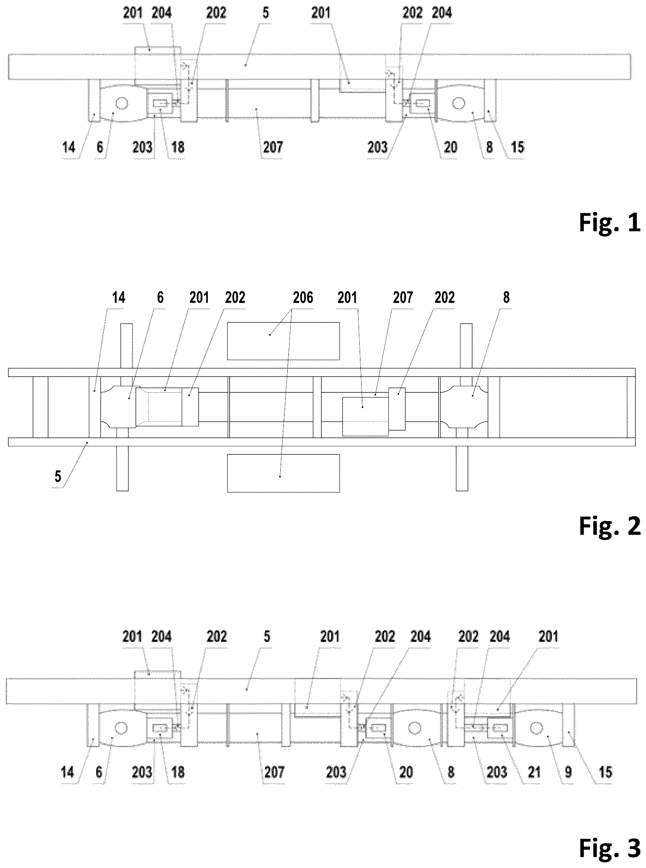

The invention will be explained in more detail using particular example embodiments shown in the drawings, of which
- fig. 1: diagram of a 4x4 chassis with driving units in side view, with the flow of torque marked out in dashed line,
- fig. 2: diagram of a 4x4 chassis with driving units in plan view,
- fig. 3: diagram of a 6x6 chassis with driving units in side view, with the flow of torque marked out in dashed line,
- fig. 4: diagram of a 6x6 chassis with driving units in plan view,
- fig. 5: diagram of a 8x8 chassis with driving units in side view, with the flow of torque marked out in dashed line,
- fig. 6: diagram of a 8x8 chassis with driving units in plan view,
- fig. 7: diagram of the propulsion in motor mode with the flow of torque marked out in dashed line and the flow of electric energy marked out in dot and dashed line, and
- fig. 8: diagram of the propulsion in generator mode with the flow of torque marked out in dashed line and the flow of electric energy marked out in dot and dashed line.

### Example Embodiment of the Invention

Advantageous embodiments of the invention will be described further on three variants of the chassis fitted with drivetrain, 4x4, 6x6 and 8x8. But as will be obvious to persons skilled in the art, the presented invention also comprises other variants, such as 4x2 and 6x4.

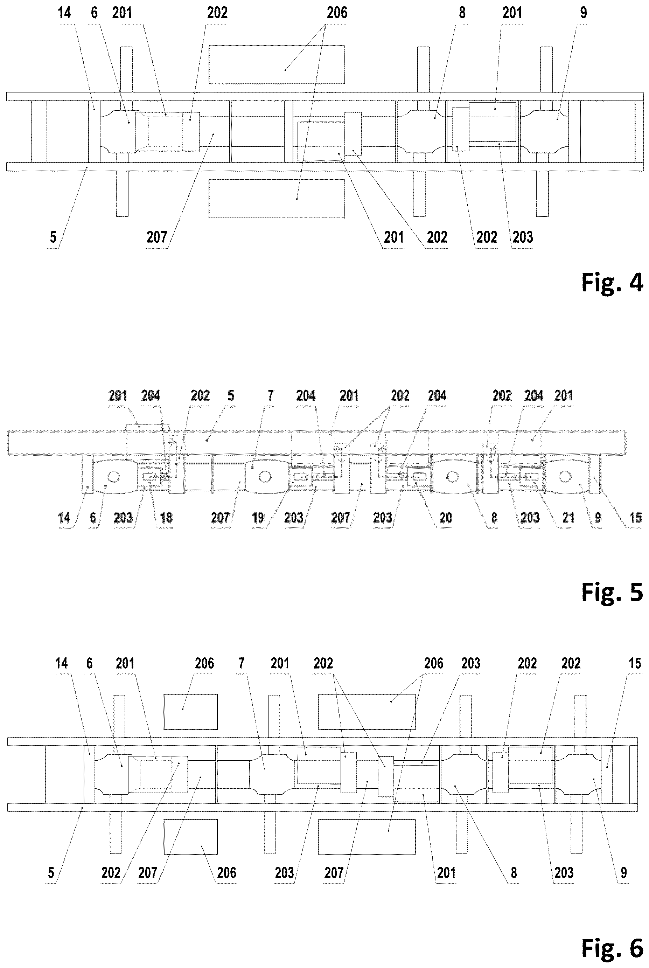

As an example, referring to fig. 5 and 6, it is clear that the 8x8 variant optionally contains frame 5 with two longitudinal beams and front crossbeam 14 and rear crossbeam 15. The chassis containing central load-carrying tube 207 as the basic load carrying element further contains first front axle 6, second front axle 7, first rear axle 8 and second rear axle 9. Individual axles 6-9 have then assigned the axis differential 18 of the first front axle 6, axis differential 19 of the second front axle 7, axis differential 20 of the first rear axle 8 and axis differential 21 of the second rear axle 9, these differentials are installed in the connecting load carrying tube 203.

The present invention is thus for clearness of the most advantageous embodiment of the present invention presented as an all-wheel drive chassis, especially for heavy offroad trucks, using drive units, which contain for each of the axles 6-9 always one electric motor 201, auxiliary gearbox 202, connecting load carrying tube 203 and axis differential 18-21. Each electric motor 201 always drives only one axle 6-9 with swinging half-axles. Such drive unit could be thus combined with other drive units and central load carrying tube 207 into a complete chassis of the required configuration, as listed above and presented on figures.

The presented technical solution of the chassis contains central load carrying tube 207 as a basic construction element common for the construction solutions of vehicles of the applicant. Central load carrying tube 207 is connected with drive units using bolted joints. The cross beams 14 and 15 are connected to the drive units in the same way. Central load carrying tube 207 could be connected to the drive unit either from the side of the auxiliary gearbox 202, or from the side of the axle 6-9. The difference between central load carrying tube 207 and connecting load carrying tube 203 is especially in the fact that axis differential 18-21 is placed in the connecting load carrying tube 203, wherein the central load carrying tube 207 has the function of connecting drive units between them. Each drive unit contains an electric motor 201, to which the auxiliary gearbox 202, connecting load carrying tube 203 and axis differential 18-21 of the respective axle 6-9 are connected in the direction of the flow of the torque transferred. Further are the axles 6-9 with swinging half axles connected.

Such drive unit is a compact ensemble, independent on possible other drive units, if arranged. Thanks to this fact, this drive unit could be used in almost any place in the automobile, in any direction, either facing the direction of travel or opposite it. This variability is enabled by the modular construction of the chassis with central load carrying tube 207, where it is possible to variably change the dimensions and thus to change the wheel bases between axles 6-9. It is also possible to easily add other axles 6-9 with drive units, and thus create various chassis combinations. Number of axles with drive units used is unlimited in theory, in reality it is limited by the ability of the automobile to manoeuvre in required space.

Presented invention is characterised by the absence of internal combustion engine, main gearbox, cardan shaft, supplementary gearbox, inter-axle differential, or divider, if used, exhaust and emission system and fuel tank and thanks to lower weight of the whole chassis, it is advantageously possible to increase the payload.

The centre of gravity also gests lower thanks to the lower installation of the drivetrain with electric motors 201, which improves driving characteristics.

Electric motors 201 for driving the chassis can work in three modes - motor, generator and recuperative.

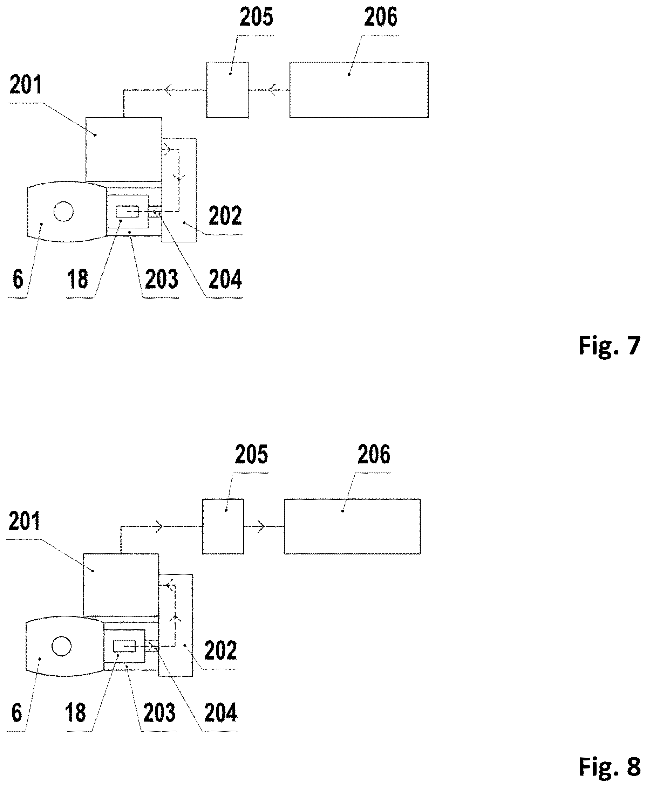

In the motor mode (fig. 7), the electric energy from battery 206 is led through inverter 205 into electric motor 201, torque from electric motor 201 is then transferred through auxiliary gearbox 202 into axle differential 18-21. From axle differential 18-21, it is transferred to the axle 6-9 and further to individual wheels. This mode will be used especially for standing starts and accelerating from stable speed.

In generator mode (fig. 8) the stable, constant speed ride of the automobile e.g. on highway is used. One or more drive units works in motor mode and the rest of drive units in the chassis work in generator mode and make use of the movement of the automobile and through the wheels, axis differential 18-21 of the axle and auxiliary gearbox 202 rotate the electric motor 201, which then generates electric energy and charges the battery 206 through the inverter 205.

Recuperative mode is used during slowing down the vehicle, when all drive units are switched into the generator mode (fig. 8) and they are primarily used to lower the speed of the vehicle, which at the same time reduces the wear on brakes and causes lower emissions of solid particles from brake lining.

Thanks to the modular, respective sectional, construction of the whole chassis, variable placement of the electric motor 201 in the direction of travel in front of or behind the auxiliary gearbox 202 is possible, taking into account optimal weight distribution, or taking into account the placement of other chassis components. This allows for almost unlimited possibilities of combining drive units with axles 6-9 and central load carrying tube 207 into a complete chassis with various numbers of axles 6-9 and variable wheel bases.

Because each electric motor 201 could function independently, it is possible to use effective control of individual electric motors 201 and switch them in any order into motor and generator mode according to current situation.

During standing start, the control electronic switches required number of electric motors 201 needed for standing start of the automobile, depending on adhesion conditions, weight of the vehicle, slope and other input parameters.

After the start and stabilisation of the speed at a constant value, the control electronic evaluates, which electric motors 201 could be switched into generator mode and thus recharge the batteries 206 during the ride.

When slowing down the vehicle, all drive units are switched to generator mode and electric motors 201 are used as primary source of brake force, together with operational brakes.

Mechanical inter-axle differential is thus replaced by control electronic, which effectively controls the rotational speed and torque of individual electric motors 201 based on current adhesion conditions.

Although the present invention was further described in connection with certain advantageous embodiments, it is not the purpose of this description to limit its scope to such described embodiments only. Conversely, the aim of this description is to comprise and cover all alternatives, modifications and equivalents of such embodiments, which belong to the subject-matter and claimed scope of the presented invention, as defined in attached claims.

Both above mentioned and further mentioned features according to the invention could be used individually as stand-alone, or in any mutual combinations, if not otherwise stated or if there is no clear contradiction in the context.

References stated in dependent claims refer to other embodiments of the subject-matter of the independent claim using the features of the respective dependent claim. But they don't mean resignation on reaching independent, objective protection for the features of these dependent claims.

The invention thus isn't limited to the embodiment described, and a person skilled in the art can embody the details of the invention using many adaptations within the scope of the attached claims.

### List of Reference Numerals

- 5: frame
- 6: 1. front axle
- 7: 2. front axle
- 8: 1. rear axle
- 9: 2. rear axle
- 14: front crossbeam
- 15: rear crossbeam
- 18: axis differential of the first front axle
- 19: axis differential of the second front axle
- 20: axis differential of the first rear axle
- 21: axis differential of the second rear axle
- 201: electric motor
- 202: auxiliary gearbox
- 203: connecting load carrying tube between the auxiliary gearbox and axle
- 204: drive shaft
- 205: inverter
- 206: battery
- 207: central load carrying tube between individual drive units

## Claims

1. Chassis, especially for heavy offroad truck, fitted with drivetrain, wherein the chassis contains central load carrying tube (207) with at least two axles (6-9) with swinging half-axles, where the chassis has at least one drive unit assigned as part of the drivetrain,
***characterised by***
the drive unit consisting of electric motor (201), with assigned auxiliary gearbox (202), connecting load carrying tube (203) and axis differential (18-21), wherein the drive unit is always assigned to only one axle (6-9).

2. Chassis according to claim 1,
***characterised by***
the electric motor (201) being through the auxiliary gearbox (202) fixedly connected to the load carrying tube (203, 207).

3. Chassis according to claim 1 or 2,
***characterised by***
electric motor (201) being oriented with its axis of rotation in the direction of travel.

4. Chassis according to some of the claims 1 to 3,
***characterised by***
electric motor (201) is placed in front of or behind the auxiliary gearbox (202) in the direction of travel.

5. Chassis according to any of the claims 1 to 4,
***characterised by***
the torque being led in steered axles (6-9) from the axis differential (18-21) into the wheel parts through cardan shafts, and in non-steered axles (6-9) using rigid shafts going through the axles' (6-9) half-shafts.

6. Chassis according to some of the preceding claims 1 to 5,
***characterised by***
individual axles (6-9) are fitted with autonomous electronic control, independent on the control of the rest of the axles (6-9).
